(19) Europäisches Patentamt
European Patent Office
Office européen des brevets

(11) Veröffentlichungsnummer: **0 136 646**
**B1**

# (12) EUROPÄISCHE PATENTSCHRIFT

(45) Veröffentlichungstag der Patentschrift: **25.01.89**

(51) Int. Cl.⁴: **C 07 D 265/22**

(21) Anmeldenummer: **84111389.7**

(22) Anmeldetag: **25.09.84**

(54) **Verfahren zur Herstellung von 5-Fluor-2-phenyl-4H-3,1-benzoxazin-4-onen.**

(30) Priorität: **30.09.83 DE 3335456**
**06.03.84 DE 3408154**

(43) Veröffentlichungstag der Anmeldung: **10.04.85 Patentblatt 85/15**

(45) Bekanntmachung des Hinweises auf die Patenterteilung: **25.01.89 Patentblatt 89/4**

(84) Benannte Vertragsstaaten: **BE CH DE FR GB IT LI**

(56) Entgegenhaltungen:
**EP-A-0 017 931**
**EP-A-0 053 247**

**HOUBEN-WEYL: "Methoden der organischen Chemie, Band V/3, 4. Auflage, 1962, Seiten 160-166, Georg Thieme Verlag, Stuttgart, DE, E. FORCHE u.a.: "Halogen-Verbindungen Fluorverbindungen, Herstellung, Reaktivität und Umwandlung"; JOURNAL OF THE AMERICAN CHEMICAL SOCIETY, Band 81, Nr. 9, 14. Mai 1959, Seiten 2674-2675, American Chemical Society, US; G.C. FINGER u.a.: "Aromatic fluorine compounds. IX. 2-Fluoropyridines"**

(73) Patentinhaber: **BASF Aktiengesellschaft, Carl-Bosch- Strasse 38, D-6700 Ludwigshafen (DE)**

(72) Erfinder: **Varwig, Juergen, Dr., Bitterstrasse 21, D-6900 Heidelberg (DE)**
Erfinder: **Hamprecht, Gerhard, Dr., Rote- Turm- Strasse 28, D-6940 Weinheim (DE)**
Erfinder: **Rohr, Wolfgang, Dr., In der Dreispitz 13, D-6706 Wachenheim (DE)**

**EP 0 136 646 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

5-Fluor-2-phenyl-4H-3,1-benzoxazin-4-one sind herbizid wirksam und zeichnen sich durch hohe Aktivität und ein breites Wirkungsspektrum aus (DE-OS-2 914 915, DE-OS-3 000 309, DE-OS-3 037 970). Typische Verbindungen dieser Art werden z. B. durch die allgemeine Formel (I) beschrieben,

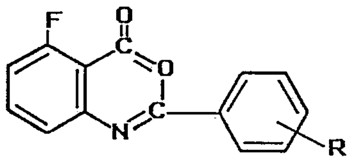

(I),

in der R Wasserstoff, Halogen, $C_1$- bis $C_3$-Alkyl, m- oder p-ständiges Trifluormethyl, Trifluormethoxy, Chlordifluormethoxy, Difluormethoxy, Tetrafluorethoxy, Trifluormethylmercapto oder Chlordifluormethylmercapto bedeutet.

Üblicherweise werden die Verbindungen (I) durch Umsetzung von 6-Fluoranthranilsäure mit entsprechend substituiertem oder unsubstituiertem Benzoylhalogenid und Cyclisierung hergestellt (vgl. oben).

Dieses Verfahren ist unwirtschaftlich, da die erforderliche 6-Fluoranthranilsäure noch nicht preiswert genug hergestellt werden kann (DE-OS-3 044 904, EP-A-55 629).

Es wurde nun ein Verfahren gefunden, das ausgeht von entsprechenden, in 5-Stellung substituierten 4H-3,1-Benzoxazin-4-onen der Formel (II)

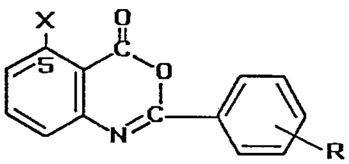

(II),

in der

X vorzugsweise Chlor oder Brom, eventuell auch Jod, Nitro, Mesyl oder Tosyl bedeutet.

Hierzu ist folgendes vorauszuschicken:

Nach Houben-Weyl, Methoden der Organischen Chemie, 4. Aufl.- Bd. V/3, S. 160 ff. läßt sich aromatisch gebundenes Chlor nur dann gegen Fluor austauschen wenn es durch zwei elektronenziehende Substituenten in o- und p-Stellung gelockert ist. Bei carbonestersubstituierten Aromaten gelingt der Halogenaustausch z. B. nur bei gleichzeitiger Anwesenheit einer zusätzlichen Nitrogruppe.

Unterstrichen wird diese Regel durch die Beobachtung, daß der Halogenaustausch auch bei 2-Chlorpyridinen nur dann gelingt, wenn das Chlor durch eine o- oder p-ständige Nitrogruppe gelockert ist. Auf diese Weise können z. B. gewisse 2- bzw. 6-Fluor-nitro-pyridine gewonnen werden (J. Amer. chem. Soc. *81*, 2674 (1959)). Das Stickstoffatom des Pyridinringes bewirkt für sich allein keine genügende Lockerung des Chlorstoms in der 2-Stellung; aus 2-Chlor- bzw. 3-Brom-pyridin kann nach allgemeinem Fachwissen kein 2-Fluor-pyridin hergestellt werden. Auch zeigt sich der geringe aktivierende Effekt einer m-Nitrogruppe darin, daß sich 2-Chlor-4-nitro-pyridin mit Kaliumfluorid weder in Dimethylformamid noch in Dimethylsulfoxid bei 160°C in die Fluorverbindung überführen läßt (Houben-Weyl, loc. cit., S. 165).

Weiterhin ist bekannt, daß o-ständige Chloratome bei dem Halogenaustausch an substituierten aromatischen Carbonsäurederivaten zu Carbonylzersetzungsreaktionen führen. So beschreiben Odinokov et al. (CA. *69*, 187977), daß 3,6-Dichlorphthalsäureanhydrid bei der Umsetzung mit Kaliumfluorid während 3 Stunden bei 190 bis 200°C in nur 54 % Ausbeute 3,6-Difluorphthalsäureanhydrid bildet, während bei einer Temperatur von 240 bis 245°C während 8 Stunden in 52 % Ausbeute unter Abspaltung von Kohlendioxid das Bislacton der α,α'-Dihydroxy-3,6,3',6'-tetrafluordiphenylmethan-2,2'-dicarbonsäure als Hauptprodukt gebildet wird. Bei noch höherer Temperatur - 330 bis 340°C - tritt nochmals Kohlendioxidabspaltung unter Bildung eines 1,4,5,6-Tetrafluoranthrachinons auf.

Es ist ferner bekannt, daß Kaliumfluorid nucleophil einerseits Sulfitester (J. Org. Chem. *31*, 842 (1966)) und Acylester (J. Chem. Soc. *1962*, 1056; J. Chem. Soc. Perkin Trans. I, *1978*, 269) unter Eliminierung der Acylgruppe angreift, daß es aber auch andererseits aromatische Carbonsäuren, z. B. Anthranilsäure leicht decarboxyliert (C.A. *42*, 4924 g). Alle diese Nebenreaktionen beeinträchtigen den Halogenaustausch von beispielsweise 5-chlorsubstituierten 2-Phenyl-4H-3,1-benzoxazin-4-on-derivaten mit Kaliumfluorid und führen zu nicht unerheblichen Mengen an Zersetzungsprodukten, wie das nachstehende Schema zeigt:

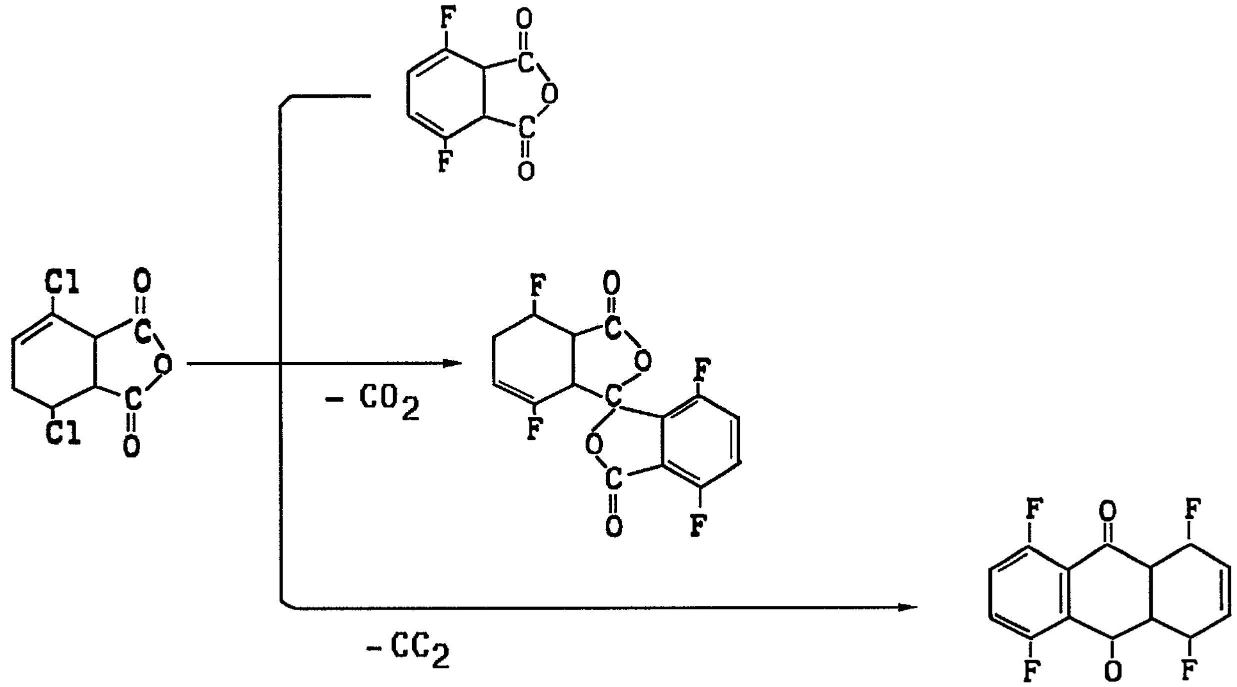


| | | % | |
|---|---|---|---|
| 5 Std. | 190 - 200°C | 54 | (a) |
| 8 Std. | 240 - 245°C | 52 | (b) |
| 2,5 Std. | 330 - 340°C | 30 | (c) |

Es wurde nun gefunden, daß man 5-Fluor-2-phenyl-4H-3,1-benzoxazin-4-one der vorstehend näher erläuterten Formel (I)

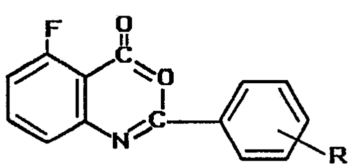

(I)

aus entsprechenden chlor-, brom- oder auch jod-, nitro-, mesyl- oder tosyl-substituierten 2-Phenyl-4H-3,1-benzoxazin-4-onen (II) erhalten kann, wenn man diese unter Wasserausschluß mit Alkalifluorid umsetzt.

Die Ausgangsverbindungen können mit stöchiometrischen oder überschüssigen Mengen an (Alkyl)ammoniumfluorid oder Alkalimetall-fluorid, bevorzugt mit Kaliumfluorid, zweckmäßig 1 bis 5 mol, vorzugsweise 1 bis 1,5 mol, insbesondere 1 bis 1,15 mol pro mol Benzoxazinon umgesetzt werden.

Die Umsetzung in Gegenwart eines Lösungsmittels verläuft mit guter Ausbeute bei 150 bis 250°C, vorzugsweise bei 180 bis 230°C, bei atmosphärischem oder wenn nötig erhöhtem Druck, absatzweise oder fortlaufend. Oberhalb dieses Temperaturbereiches verschlechtern sich die Ausbeuten. Als Lösungsmittel ist z. B. Dimethylformamid, Dimethylsulfoxid, ein Polyethylenglykoldialkylether, Acetonitril, Hexamethylphosphorsäuretriamid, ein offenkettiges oder cyclisches Sulfon, wie z. B. Sulfolan geeignet. Wenn man ohne ein Lösungsmittel arbeiten will, so muß jedoch eine Temperatur oberhalb von 250°C, z. B. 300 bis 500°C, angewandt werden, die in diesem Falle entgegen der Erwartung von den Ausgangs- und Zielstoffen ertragen wird.

Nach einer besonderen Ausgestaltung des Verfahrens führt man die Umsetzung in Gegenwart eines Metalljodids und/oder von Jod durch, die Temperatur bei der Umsetzung beträgt 160 bis 280°C, insbesondere 180 bis 260°C und man setzt das Metalljodid bzw. Jod während der Umsetzung zu.

Beim Arbeiten in einem Lösungsmittel ist der Zusatz eines Löslichkeitsvermittlers - besonders bei Anwendung von Kaliumfluorid - sehr zu empfehlen. Geeignet sind z. B. Kronenether, Polyethylenglykole oder deren Derivate. Relativ gut löslich ist Caesiumfluorid. Gewöhnlich wird dagegen bevorzugt Kalium- oder Ammoniumfluorid verwendet und als Lösungsmittel ein Sulfon, z. B. Sulfolan. Wenn man Caesiumfluorid verwendet, kann man auf einen Vermittler verzichten und - aus wirtschaftlichen Gründen - dieses mit einem anderen Fluorid strecken bzw. regenerieren.

Eine weitere Verbesserung des Verfahrens wird dadurch erzielt, daß man die Umsetzung in Gegenwart eines Metalliodids oder von Jod vornimmt, die mit dem Halogenaustausch einhergehende Zersetzungsreaktionen des

Heterocyclus inhibieren.

Nach einer weiteren besonderen Ausgestaltung des Verfahrens führt man die Umsetzung im wesentlichen ohne Lösungsmittel, in Gegenwart eines Metalljodids zwischen 300 und 400°C durch.

Die Menge an Jodid bzw. Jod beträgt z. B. 1 bis 10, insbesondere 0,1 bis 5 mol.-%, bezogen auf Benzoxazinon.

Für das erfindungsgemäße Verfahren sind wasserfreie Reaktionsbedingungen Vorraussetzung.

Eine vorherige intensive Trocknung der Ausgangsmaterialien und gegebenenfalls des Lösungsmittels ist daher wichtig für die technische Durchführbarkeit. Destillation der Lösungsmittel oder Zusatz wasserentziehender Mittel und insbesondere intensive Trocknung des Fluorierungsmittels und dessen feine Verteilung im Reaktionsmedium sind wünschenswert.

Der Trocknung muß besondere Aufmerksamkeit gewidmet werden, wobei es wegen der Gewinnung der Zielprodukte vorteilhaft ist, wenn man Trocknungsmittel anwendet, die keine unmverdampfbaren Rückstände hinterlassen. Halogenide der schwefligen Säure oder Kohlensäure, insbesondere Thionylchlorid werden bevorzugt.

Zwar genügt es oft, das Fluorsalz durch entsprechende Vorbehandlung zu trocknen, jedoch ist es am sichersten, wenn die Umsetzung in Gegenwart des Trocknungsmittels vorgenommen wird. Die Vorbehandlung, wenn man sich hierauf beschränkt, kann bei einer Temperatur unter 120°C vorgenommen werden.

Allgemein kann man wie folgt verfahren:

Das trockene Benzoxazinon, gegebenenfalls Jodid bzw. Jod, und gut getrocknetes und fein gepulvertes Kaliumfluorid werden bei ausreichend hoher Temperatur je nach Verfahrensweise mit oder ohne Lösungsmittel, mit oder ohne Katalysator während etwa 2 Minuten bis 24 Stunden umgesetzt, der anorganische unlösliche Rückstand wird abgetrennt und kann nachgewaschen werden. Bei Verwendung eines Lösungsmittels, wie z. B. Sulfolan, wird dieses abdestilliert. Das Produkt kann gegebenenfalls z. B. mit Essigsäure und/oder Acetanhydrid, Ethanol, Ether, Wasser, Aceton oder Methylenchlorid gewaschen bzw. umkristallisiert werden. Eine besonders bevorzugte Reinigungsmethode stellt die Destillation des Produktes unter vermindertem Druck und bei erhöhter Temperatur dar.

Nach dem neuen Verfahren werden als Ausgangsstoffe vor allem 5-chlor- bzw. 5-bromsubstituierte 2-Phenyl-4H-3,1-benzoxazin-4-one der Formel II, das sind beispielsweise 5-Chlor-2-phenyl-4H-3,1-benzoxazin-4-on, 5-Chlor-2-(3'-trifluoromethyl-phenyl)-4H-3,1-benzoxazin-4-on, 5-Chlor-2-(3'chlordifluormethoxy-phenyl)-4H-3,1-benzoxazin-4-on, 5-Chlor-2-(3'trifluormethoxy-phenyl)-4H-3,1-benzoxazin-4-on, 5-Chlor-2-(3'difluormethoxy-phenyl)-4H-3,1-benzoxazin-4-on, 5-Chlor-2-(3'-tetrafluorethoxy-phenyl)-4H-3,1-benzoxazin-4-on, 5-Chlor-2-(3'-trifluormethylmercaptophenyl)-4H-3,1-benzoxazin-4-on, 5-Chlor-2-(3'-chlordifluormethylmercapto-phenyl)-4H-3,1-benzoxazin-4-on, 5-Chlor-2-(4'-chlordifluormethoxy-phenyl)-4H-3,1-benzoxazin-4-on, 5-Chlor-2-(4'-chlordifluormethylmercapto-phenyl)-4H-3,1-benzoxazin-4-on, 5-Chlor-2-(4'-tetrafluorethoxy-phenyl)-4H-3,1-benzoxazin-4-on oder deren entsprechende 5-Bromanaloge benötigt, die zweckmäßig in einer ersten Stufe in Gegenwart eines aliphatischen Sulfons bei bis zu 120°C mit Thionylchlorid oder Phosgen vorbehandelt werden. Im allgemeinen ist für die Vorbehandlung eine Temperatur von unter 120°C ausreichend. Lösungsmittel und Säurehalogenid brauchen danach im Falle der Durchführung der Hauptreaktion in einem aliphatischen Sulfon nicht entfernt zu werden.

Als aliphatische Sulfone sind z. B. die Verbindungen der Formel (III)

$$R^1\text{-}\overset{\overset{\displaystyle O}{\|}}{\underset{\underset{\displaystyle O}{\|}}{S}}\text{-}R^2 \qquad \text{(III)}$$

geeignet, in der $R^1$ und $R^2$ gleich oder verschieden sind und jeweils einen aliphatischen Rest, vorzugsweise einen Alkylrest mit 1 bis 8, insbesondere 1 bis 4 Kohlenstoffatomen oder $R^1$ und $R^2$ zusammen einen Alkylenrest mit 4 oder 5 Kohlenstoffatomen bedeuten. Gut geeignete Lösungsmittel der genannten Art sind beispielsweise Dimethylsulfon, Diethylsulfon, Dipropylsulfon, Diisopropylsulfon, Dibutylsulfon, Diisobutylsulfon, Methylethylsulfon, Tetramethylensulfon (Sulfolan) und Pentamethylensulfon, von denen Sulfolan bevorzugt ist. Im allgemeinen verwendet man das Lösungsmittel in bis zu 10-fachem Gewichtsüberschuß, meist beträgt sein Gewichtsanteil im Reaktionsgemisch 100 bis 400 Gewichtsprozent, bezogen auf Ausgangsstoff II. Das Säurechlorid wird zweckmäßig in einer Menge von 1 bis 20 Gewichtsprozent, vorzugsweise von 2 bis 12 Gewichtsprozent, bezogen auf das Sulfon eingesetzt.

Als Katalysatoren sind für die Vorbehandlung z. B. N,N-disubstituierte Carbonamide mit 3 bis 10 Kohlenstoffatomen geeignet, wie N,N-Dimethylformamid, N,N-Dimethylacetamid, N,N-Diethylformamid, N,N-Di-n-propylacetamid, N-Methyl-N-ethyl-formamid oder N,N-Di-isopropylacetamid. Der Katalysator wird zweckmäßig in einer Menge von 0,2 bis 2 Gewichtsprozent bezogen auf das Säurehalogenid eingesetzt.

Man kann, wenn nötig, auch das Alkalifluorid mit einem der obengenannten Säurehalogenide auf gleiche Weise, gegebenenfalls in einem inerten, bis 120° siedenden Lösungsmittel vorbehandeln. Zweckmäßig suspendiert man das Alkalifluorid in dem überschüssigen Lösungsmittel und gibt dann das Säurehalogenid, zweckmäßig in einer Menge von bis zu 100 Gewichtsprozent, vorzugsweise von 2 bis 30 Gewichtsprozent, bezogen auf das Alkalifluorid, hinzu. Anschließend rührt man unter Erwärmen, z. B. bis zu 120°C, zweckmäßig zwischen 50 bis 110°C, insbesondere bei 70 bis 100°C nach und engt dann bei 10 bis 30 mbar ein. Die Behandlung dauert gewöhnlich nicht mehr als 2 Stunden. Das so behandelte Kaliumfluorid gibt man dann zu einem Gemisch von unbehandeltem oder vorbehandeltem Ausgangsstoff II mit dem aliphatischen Sulfon. Man kann jedoch auch das

Gemisch des Ausgangsstoffes II mit dem aliphatischen Sulfon - wie beschrieben - mit einem Säurechlorid vorbehandeln und gibt dann, nach dem Ende der Gasentwicklung, trockenes, unbehandeltes Alkalifluorid, Metalljodid und/oder Jod hinzu.

An sich können der Ausgangsstoff (Benzoxazinon; II), das Sulfon, das Metalljodid und das Säurehalogenid in beliebiger Reihenfolge miteinander vermischt werden. Zweckmäßig vermischt man jedoch zunächst bei 30 bis 40°C den Ausgangsstoff II mit dem Metalljodid und dem Sulfon III und gegebenenfalls mit einem Katalysator, fügt dann unter Rühren das Säurehalogenid zu und erhitzt zweckmäßig so lange, bis keine Gasentwicklung mehr beobachtet wird. Man kann überschüssiges Säurehalogenid z. B. durch Einblasen von Stickstoff o.ä. oder durch Anwendung von vermindertem Druck entfernen.

Vorteilhaft gibt man jedoch das Metalljodid und insbesondere elementares Jod - aufgrund seiner Flüchtigkeit - erst nach der Säurehalogenidbehandlung des Ausgangsstoffes II in dem Sulfon zu.

Nach einer besonders vorteilhaften Verfahrensweise vermischt man die sorgfältig getrockneten Ausgangsstoffe II, Kaliumfluorid, Sulfolan, Metalljodid und/oder Jod mit dem Säurehalogenid und führt dann die Umsetzung durch. Man kann jedoch auch bei dieser Verfahrensweise Jodid bzw. Jod erst später im Verlauf der Umsetzung, spätestens allerdings nach der Hälfte der Umsetzung, d. h. Reaktionszeit, zugeben.

Zweckmäßig soll schließlich - zur Vermeidung unnötiger Fluoridverluste - die noch verbliebene Menge des Säurehalogenids 1 bis 5 Molprozent bezogen auf das Alkalifluorid bei der eigentlichen Umsetzung nicht übersteigen. In der Regel genügt die nach der Vorbehandlung des Gemisches aus Ausgangsstoff II und aliphatischem Sulfon nach Einblasen eines Inertgases oder Druckverminderung auf 10 bis 30 mbar noch vorhandene Restmenge an Säurehalogenid zur Behandlung des Alkalifluorids, das zugesetzt wird, um die eigentliche Umsetzung in Gang zu bringen.

Zur eigentlichen Umsetzung ist, soweit nicht schon geschehen, noch folgendes zu bemerken.

Der Halogenaustausch verläuft bereits ohne Katalysator mit hoher Geschwindigkeit. Er kann jedoch beim Arbeiten in einem Lösungsmittel durch Zugabe eines Kronenethers oder eines sogenannten Cryptanden beschleunigt werden. Darunter versteht man organische Komplexliganden, die insbesondere gut zur Bindung von Alkali dienen. Kronenether sind ringförmig verknüpfte neutrale Ethylenglykolether. Die Cryptanden liefern eine dreidimensionale Umhüllung. Die Herstellung solcher Stoffe ist in der Zeitschrift Kontakte (1977), Seiten 11 bis 31 und 36 bis 48 beschrieben. Als Kronenether seien z. B. die folgenden Verbindungen genannt:

12-Krone-4; 2,4,6,8-Methyl-12-krone-4; 14-Krone-4; Dibenzo-14-krone-4; Dibutyl-benzo-14-krone-4; Dicyclohexyl-14-krone-4; 15-Krone-5; 1,2-Benzo-15-krone-5; 1,2-Butylbenzo-15-krone-5; 1,2-Cyclohexyl-15-krone-5; Dibenzo-15-krone-5; 16-Krone-5; Dibenzo-16-krone-5; 18-Krone-5; Dibenzo-18-krone-5; 18-Krone-6; Benzo-18-krone-6; Cyclohexyl-18-krone-6; Dibenzo-18-krone-6; Dicyclohexyl-18-krone-6; Tribenzo-18-krone-6; Dinaphtho18-krone-6; 19-Krone-6; Dibenzo-19-krone-6; 20-Krone-7; Dibenzo-5-oxy-20-krone-7; 21-Krone-7; Dibenzo-21-krone-7; Dicyclohexyl-21-krone-7; 24-Krone-8; Dibenzo-24-krone-8; Dicyclohexyl-24-krone-8; Tetrabenzo-24-krone-8; 30-Krone-10; 40-Krone-20; Aza-18-krone-6; Dibenzo-aza-18-krone-6; Diaza-18-krone-6; Dibenzo-diaza-18-krone-6; 1,4-Dithia-15-krone-5; 1,4-Dithia-18-krone-6; 1,7-Dithia-benzo-18-krone-6; 1,10-Dithia-benzo-18-krone-6 und 1,7,10,16-Tetrathia-18-krone-6.

Die Katalysatoren werden zweckmäßig in einer wirksamen Menge von bis zu 0,5, insbesondere 0,1 bis 0,3 Molprozent je mol Ausgangsstoff II eingesetzt.

Nach der Umsetzung, die etwa nach 4 bis 12 Stunden beendet ist, arbeitet man auf an sich übliche Weise, z. B. durch Filtration, Destillation von Filtrat und Waschfiltraten, Waschen des Festgutes usw. auf.

Für das Arbeiten ohne Lösungsmittel ist das Folgende von Bedeutung:

Zweckmäßig legt man den Ausgangsstoff II in geschmolzenem Zustand vor und mischt dann das trockene Fluorsalz sowie gegebenenfalls ein Metalljodid hinzu. Man kann jedoch auch über geeignete Dosiervorrichtungen den Ausgangsstoff II, das Fluorsalz sowie gegebenenfalls ein Metalljodid gleichmäßig zuführen und miteinander vermischen. Führt man die Umsetzung oberhalb des Siedepunktes der Endstoffe I durch, so legt man zweckmäßig das Fluorsalz beispielsweise auf einem Träger oder in einem Wirbelbett vor und dosiert dann den Ausgangsstoff II gasförmig hinzu, wobei der Endstoff destillativ aus dem Reaktionsraum entfernt wird.

Ist das Fluorsalz noch wasserhaltig, so wird es zunächst unter den Reaktionsbedingungen, gegebenenfalls unter vermindertem Druck, getrocknet und dann der Ausgangsstoff II zudosiert.

Werden das Fluorsalz und der Ausgangsstoff II gleichmäßig zudosiert, so ist nach anfänglicher Durchmischung ein weiteres Rühren der Reaktionsmischung zur Beendigung der Reaktion nicht erforderlich. Die Reaktion kann daher auch nach der Art eines Backprozesses oder in einem Röhrenreaktor durchgeführt werden.

Die Reaktionstemperatur kann gewissen apparativen Gegebenheiten und Heizmöglichkeiten angepaßt werden, soll jedoch zweckmäßig mindestens 290°C bis 300°C betragen. In diesem unteren Temperaturbereich müssen entsprechend lange Reaktionszeiten gewählt werden, z. B. 18 bis 24 Stunden bei 300°C. In einem mittleren Temperaturbereich, z. B. 380°C, beträgt die Reaktionsdauer etwa 25 bis 35 Minuten und verkürzt sich dementsprechend bei noch höheren Temperaturen.

In einer vorteilhaften Ausgestaltung des erfindungsgemäßen Verfahrens destilliert man den Endstoffe im Maße seiner Entstehung direkt aus dem Reaktionsgemisch ab.

Im allgemeinen, z. B. bei der Zudosierung des Ausgangsstoffes II in geschmolzenem Zustand, wählt man eine Reaktionstemperatur von 320 bis 410°C; wird der Ausgangsstoff II gasförmig zugegeben, so kann man eine noch höhere Reaktionstemperatur wählen, wobei man die Umsetzung drucklos oder aber bei Unterdruck durchführt.

Nach der Umsetzung arbeitet man auf an sich übliche Weise, z. B. durch Destillation oder Waschen des Festgutes zur Entfernung der Metallsalze auf.

**Beispiel 1**

6,0 kg 5-Chlor-2-phenyl-4H-3,1-benzoxazin-4-on und 2,3 kg gut getrocknetes und fein gepulvertes Kaliumfluorid werden in 18 l trockenem Sulfolan 11 Stunden bei 200 bis 220°C gerührt. Die Mischung wird bei 100°C filtriert, nachgewaschen und das Lösungsmittel abdestilliert.

Der Rückstand wird bei 200°C und 5 mbar destilliert und mit Wasser gewaschen. Man erhält 3 215 g 5-Fluor-2-phenyl-4H-3,1-benzoxazin-4-on mit einem Schmelzbereich von 162 bis 164°C.

Unter anderem lassen sich aus der entsprechenden 5-Chlorverbindung in entsprechender Weise herstellen: 5-Fluor-2-(3'-Trifluormethyl-phenyl)-4H-3,1-benzoxazin-4-on, 5-Fluor-2-(4'-trifluormethyl-phenyl)-4H-3,1-benzoxazin-4-on, 5-Fluor-2-(3'-fluorphenyl)-4H-3,1-benzoxazin-4-on, 5-Fluor-2(3'-chlorphenyl)-4H-3,1-benzoxazin-4-on, 5-Fluor-2-(4'-trifluormethoxy-phenyl)-4H-3,1-benzoxazin-4-on, 5-Fluor-2-(3'-trifluormethoxy-phenyl)-4H-3,1-benzoxazin-4-on, 5-Fluor-2-(3'-difluorchlormethoxy-phenyl)-4H-3,1-benzoxazin-4-on, 5-Fluor-2-(4'-difluorchlormethoxy-phenyl)-4H-3,1-benzoxazin-4-on, 5-Fluor-2-(4'-tetrafluorethoxy-phenyl)-4H-3,1-benzoxazin-4-on, 5-Fluor-2-(3'-tetrafluorethoxy-phenyl)-4H-3,1-benzoxazin-4-on.

**Beispiel 2**

Zu einer Mischung von 20 kg 5-Chlor-2-phenyl-4H-3,1-benzoxazin-4-on in 68 kg Sulfolan werden bei 40°C unter Rühren 4 kg Thionylchlorid gegeben. Das Gemisch wird auf 90°C erwärmt und 45 Minuten bei dieser Temperatur bis zum Ende der Gasabspaltung gerührt. Nun wird innerhalb von 12 Minuten überschüssiges Thionylchlorid im Wasserstrahlvakuum abgezogen, wobei schließlich eine Temperatur von 100°C erreicht wird. Bei 100°C werden nun 6,8 kg Kaliumfluorid eingetragen. Unter gaschromatographischer Kontrolle des Reaktionsverlaufs rührt man das Gemisch während 12 Stunden bei 215°C, bis ein Umsetzungsgrad von 96 % erreicht ist.

Nach dem Abkühlen auf 40°C, Absaugen und Waschen mit Methylenchlorid werden 8,3 kg überwiegend anorganische Salze abgetrennt. Das Filtrat wird am Rotationsverdampfer bei 50°C/12 mbar von Methylenchlorid befreit und dann durch Destillation bei 82 bis 87°C/0,3 mbar von Sulfolan befreit. Der Rückstand wird nun über eine beheizte Brücke bei einer Badtemperatur von bis zu 220°C/0,3 mbar destilliert, dann mit Wasser verrieben, abgesaugt und getrocknet. Man erhält 13,25 kg eines fast farblosen Produktes mit einem Schmelzpunkt von 154 bis 160°C bestehend aus 12,75 kg 5-Fluor- und 0,5 kg 5-Chlor-2-phenyl-4H-3,1-benzoxazin-4-on.

Das abdestillierte Sulfolan enthält noch 0,3 kg 5-Fluor-2-phenyl-4H-3,1benzoxazin-4-on. Die Gesamtausbeute an Fluorverbindung beträgt somit 13 kg (über 65 % der rechnerisch möglichen Menge). Für Zwecke des Pflanzenschutzes ist eine weitere Reinigung nicht erforderlich, da die entsprechende Chlorverbindung, die weniger wirksam ist, nicht stört.

**Beispiel 3**

a) Zu einer Mischung von 500 g Kaliumfluorid in 1 kg 1,2-Dichlorethan läßt man 50 g Thionylchlorid zulaufen und rührt 25 Minuten bei 80°C. Man entfernt das Lösungsmittel und überschüssiges Thionylchlorid am Rotationsverdampfer bei 15 mbar. Das zurückbleibende Kaliumfluorid enthält 32,5 % Fluor (ber. 32,7) und 0,3 % Chlor.
b) 8 g des so behandelten Kaliumfluorids gibt man unter Rühren bei 50°C zu einer Mischung von 75 g Sulfolan und 20 g 5-Chlor-2-phenyl-4H-3,1-benzoxazin-4-on und rührt dann 11 Stunden bei 220°C. Nach der Aufarbeitung gemäß Beispiel 2 erhält man 9,6 g überwiegend anorganische Salze, 73 g Sulfolan, 14 g fast farbloses Destillat und 4 g Destillationsrückstand. Das Destillat wird mit Wasser verrieben, abgesaugt und getrocknet, wobei man ein Gemisch aus 13 g 5-Fluor- und 0,52 g 5-Chlor-2-phenyl-4H-3,1-benzoxazin-4-on mit einem Schmelzpunkt von 156 bis 160°C erhält. In dem abdestillierten Sulfolan befinden sich weitere 0,24 g 5-Fluor-2-phenyl-4H-3,1-benzoxazin-4-on; die Gesamtausbeute beträgt 13,24 g (70,7 % d. Th.).

**Beispiel 4**

Eine Mischung von 20 g 5-Chlor-2-phenyl-4H-3,1-benzoxazin-4-on, 68 g Sulfolan, 0,8 g Natriumjodid und 5 g Thionylchlorid wird 40 Minuten bei 95 bis 100°C gerührt und dann für 20 Minuten bei 98°C auf einen verminderten Druck von 26 mbar gebracht. Unter einem Stickstoffpolster werden dann 6,76 g thionylchloridbehandeltes Kaliumfluorid (Beispiel 3a) zugegeben und 5 Stunden bei 220°C gerührt. Anschließend werden nochmals 1,24 g thionylchloridbehandeltes Kaliumfluorid zugegeben und weitere 7,5 Stunden bei 220°C unter gaschromatographischer Kontrolle des Reaktionsverlaufs gerührt, bis ein Umsetzungsgrad von 97 % erreicht ist.

Das Reaktionsgemisch wird mit Methylenchlorid verdünnt und vom überwiegend anorganischen Niederschlag abgetrennt (10,2 g). Das Filtrat wird am Rotationsverdampfer bei 50°C/12 mbar vom Methylenchlorid befreit und anschließend in einer Destillationsapparatur bei 81 bis 86°C/0,2 mbar vom Sulfolan (63,7 g) befreit. Der Rückstand wird nun über eine beheizte Brücke bei einer Badtemperatur von bis zu 220°C/0,2 mbar destilliert, wobei 19 g fast farbloses Produkt mit einem Rest Sulfolan übergehen. Nach dem Waschen mit Wasser, Absaugen und Trocknen erhält man 14,9 g Produkt mit einem Schmelzpunkt von 156 bis 161°C, bestehend aus 14,4 g 5-Fluor- und 0,5 g 5-Chlor-2-phenyl-4H-3,1-benzoxazin-4-on. Das abdestillierte Sulfolan enthält noch 0,29 g 5-Flu-

or-2-phenyl-4H-3,1-benzoxazin-4-on; die gebildete Gesamtmenge an Fluorverbindung beträgt somit 14,7 g, d. h. 78,5 % der berechneten Menge. Das wäßrige Waschfiltrat wird eingeengt, wobei 3,8 g 99 % reines (GC) Sulfolam zurückgewonnen werden.

**Beispiel 5**

Eine Mischung von 20 g 5-Chlor-2-phenyl-4H-3,1-benzoxazin-4-on, 68 g Sulfolan (über Caliumhydrid destilliert), 6,76 g Kaliumfluorid (über Nacht bei 150°C im Vakuumschrank getrocknet), 0,25 g Jod und 0,05 g Thionylchlorid wird 5,5 Stunden bei 220°C gerührt. Amschließend werden nochmals 1,24 g Kaliumfluorid und 0,25 g Jod zugegeben und weitere 7,5 Stunden bei 220°C gerührt. Nach der Aufarbeitung gemäß Beispiel 1 erhält man 10 g überwiegend anorganische Salze, 64,5 g Sulfolan und ein Gemisch aus 13,9 g 5-Fluor- und 0,6 g 5-Chlor-2-phenyl-4H-3,1-benzoxazin-4-on vom Fp. 155 bis 159°C. In dem abdestillierten Sulfolan befinden sich weitere 0,28 g 5-Fluor-2-phenyl-4H-3,1-benzoxazin-4-on; Gesamtmenge: 14,2 g, d. h. 75,8 % der berechneten Menge.

**Beispiel 6**

Ein Gemisch von 20 g 5-Chlor-2-phenyl-4H-3,1-benzoxazin-4-on, 68 g Sulfolan und 5 g Thionylchlorid wird 30 Minuten bei 100°C gerührt und dann während 30 Minuten bei 110°C/19 mbar evakuiert. Unter einem Stickstoffpolster werden 6,35 g thermisch getrocknetes Kaliumfluorid und 0,066 g Thionylchlorid zugegeben und 15 Minuten bei 240°C gerührt. Nach Zugabe von 0,25 g Jod wird noch 3,5 Stunden bei 240 C gerührt.

Nach der Aufarbeitung des Reaktionsgemisches gemäß Beispiel 1 erhält man 8,0 g überwiegend anorganische Salze, 67,5 g Sulfolan und ein Gemisch aus 13,1 g 5-Fluor- und 0,7 g 5-Chlor-2-phenyl-4H-3,1-benzoxazin-4-on vom Fp. 155 bis 159°C. In dem abdestillierten Sulfolan befinden sich weitere 0,49 g 5-Fluor-2-phenyl-4H-3,1-benzoxazin-4-on; Gesamtmenge: 13,6 g, entspr. 72,7 %.

**Beispiel 7**

Eine Mischung von 20 g 5-Chlor-2-phenyl-4H-3,1-benzoxazin-4-on, 68 g Sulfolan und 5 g Thionylchlorid wird 30 Minuten bei 100°C gerührt und dann bis 102°C/22 mbar während 30 Minuten evakuiert. Unter einem Stickstoffpolster gibt man 6,35 g thionylchloridbehandeltes Kaliumfluorid und 0,25 g Jod zu und rührt dann 2 Stunden bei 260°C.

Nach der Aufarbeitung des Reaktionsgemisches gemäß Beispiel 1 erhält man 7,7 g überwiegend anorganische Salze, 66 g Sulfolan und ein Gemisch aus 12,7 g 5-Fluor- und 0,6 g 5-Chlor-2-phenyl-4H-3,1-benzoxazin-4-on vom Fp. 155 bis 159°C neben einem Destillationsrückstand von 4,6 g. In dem abdestillierten Sulfolan befinden sich weitere 0,41 g 5-Fluor-2-phenyl-4H-3,1-benzoxazin-4-on; Gesamtmenge: 13,1 g entspr. 70 %.

**Beispiel 8**

Eine Mischung von 200 g 5-Chlor-2-phenyl-4H-3,1-benzoxazin-4-on, 649 g Sulfolan und 60 g Thionylchlorid wird 50 Minuten bei 100°C gerührt und dann während 40 Minuten bis 105°C/28 mbar evakuiert. Unter einem Stickstoffpolster gibt man 63 g thionylchloridbehandeltes Kaliumfluorid und 1 g Jod zu und rührt dann bei 220°C. Nach 4 1/4 Stunden gibt man nochmals 13,5 g Kaliumfluorid (Gesamtmenge 76,5 g) sowie 2 g Jod und nach insgesamt 6 Stunden nochmals 0,5 g Jod (Gesamtmenge 3,5 g) hinzu; die Reaktionszeit beträgt insgesamt 12 Stunden. Das Reaktionsgemisch wird bei 75°C mit 1,2-Dichlorethan verdünnt und gemäß Beispiel 4 aufgearbeitet. Man erhält 96 g überwiegend anorganisches Salz, 640 g Sulfolan und ein Gemisch aus 152,9 5-Fluor- und 5,1 g 5-Chlor-2-phenyl-4H-3,1-benzoxazin4-on vom Fp. 157 bis 161°C neben einem Destillationsrückstand von 19,8 g. Im dem abdestillierten Sulfolan befinden sich weitere 3,2 g 5-Fluor-2-phenyl-4H-3,1-benzoxazin-4-on; Gesamtmenge: 156,1 g entspr. 83,4 %.

**Beispiel 9**

In einem auf 260°C erhitzten und mit Stickstoff gespülten Kolben werden unter Rühren 50 g 5-Chlor-2-phenyl-4H-3,1-benzoxazin-4-on und 13,53 g trockenes Kaliumfluorid eingeführt. Das Reaktionsgemisch wird in 4 Minuten auf 380°C erhitzt und unter gaschromatographischer Kontrolle des Reaktionsverlaufs 25 Minuten nachgerührt. Zur Aufarbeitung wird in Methylenchlorid aufgenommen und der anorganische Niederschlsg abgesaugt. Das Filtrat wird am Rotationsverdampfer bei 50°C/14 mbar vom Methylenchlorid befreit und über eine beheizte Brücke bei einer Badtemperatur von bis zu 230°C/0,3 mbar destilliert. Man erhält 38,1 g eines fast farblosen Produkts mit einem Schmelzpunkt von 160 bis 162°C, bestehend aus 37,68 g (80,5 % d. Th.) 5-Fluor- und 0,42 g (0,8 % d. Th.) 5-Chlor-2-phenyl-4H-3,1-benzoxazin-4-on.

**Beispiel 10**

Unter gleichen Reaktionsbedingungen wie in Beispiel 9, jedoch unter Verwendung von 11,95 g des nach Beispiel 3a vorbehandelten Kalumfluorids und Rühren während 27 Minuten bei 380°C erhält man nach der Aufarbeitung 40,6 g eines fast farblosen Destillats vom Fp. 150 bis 156°C, bestehend aus 36,13 g (77,2 % d. Th.) 5-Fluor- und 4,47 g (8,9 % d. Th.) 5-Chlor-2-phenyl-4H-3,1-benzoxazin-4-on.

**Beispiel 11**

Eine Mischung aus 20 g 5-Chlor-2-phenyl-4H-3,1-benzoxazin-4-on und 6,27 g des nach Beispiel 3a vorbehandelten Kaliumfluorids wird 21 Stunden bei 300°C gerührt. Nach der Aufarbeitung gemäß Beispiel 9 erhält man 15,6 g eines fast farblosen Destillats vom Fp. 155 bis 160°C, bestehend aus 14,85 g (79,3 % d. Th.) 5-Fluor-2-phenyl-4H-3,1-benzoxazin-4-on und 0,75 g (3,7 % d. TH.) der Ausgangsverbindung.

**Beispiel 12**

44 g 5-Chlor-2-phenyl-4H-3,1-benzoxazin-4-on werden unter einem Stickstoffpolster während 3 Minuten bei 350°C mit 11,15 g trockenem Kaliumfluorid vermischt und dann 1 1/2 Stunden bei 350°C gehalten. Nach der Aufarbeitung gemäß Beispiel 9 erhält man 35,6 g eines fast farblosen Destillats vom Fp. 158 bis 160°C, bestehend aus 34,21 g (82,9 % d. Th.) 5-Fluor-2-phenyl-4H-3,1-benzoxazin-4-on und 1,39 g (3,2 % d. Th.) der 5-Chlor-Verbindung.

**Beispiel 13**

12,4 g Kaliumfluorid werden während 20 Minuten bei 330°C/14 mbar in einem Rührapparat getrocknet. Nach Belüftung mit Stickstoff gibt man bei 290°C 50 g 5-Chlor-2-phenyl-4H-3,1-benzoxazin-4-on unter Rühren hinzu und erhitzt auf 330°C. Es wird zunächst 10 Minuten bei dieser Temperatur gerührt und dann ohne weitere Rührung 1 1/2 Stunden bei 330°C gehalten. Über eine zwischengeschaltete - von außen beheizte - Kolonne mit Destillationsaufsatz vermindert man den Druck auf 212 mbar und destilliert unter Rückfluß kontinuierlich die leichter siedende Komponente bei 302 bis 284°C und einer Badtemperatur von 345°C ab. Die Innentemperatur fällt dabei zeitweise bis auf 315°C; die Dauer der Destillation, die in die Gesamtreaktionszeit miteingeht, wird auf 1 1/4 Stunden eingestellt, wobei der Druck gegen Schluß der Umsetzung auf 14 mbar zurückgenommen wird. Man erhält 43,4 g eines fast farblosen Destillats vom Fp. 147 bis 158°C bestehend aus 38,1 g (81,3 % d.Th.) 5-Fluor-2-phenyl-4H-3,1-benzoxazin-4-on und 5,2 g (10,4 % d. Th.) der 5-Chlor-Verbindung. Der pulverförmige, graue Destillationsrückstand (18,3 g) wird durch Waschen mit Wasser von den anorganischen Salzen befreit und getrocknet, wobei 3,2 g eines braunen Pulvers als Rückstand verbleiben.

**Beispiel 14**

Eine Mischung aus 50 g 5-Chlor-2-phenyl-4H-3,1-benzoxazin-4-on und 12,4 g Kaliumfluorid wird innerhalb 14 Minuten durch ein in einem elektrischen Röhrenofen auf 420 bis 430°C beheiztes Rohr geleitet und dann aus einer angeschlossenen Destillationsapparatur bei 240 bis 244°C C/15 mbar destilliert. Man erhält 39,5 g eines fast farblosen Destillats vom Fp. 144 bis 156°C, bestehend aus 35,15 g (75 % d. Th.) 5-Fluor-2-phenyl-4H-3,1-benzoxazin-4-on und 4,35 g ( 8,7 % d. Th.) des nicht umgesetzten Ausgangsstoffes.

**Beispiel 15**

4,2 g geschmolzenes 5-Chlor-2-phenyl-4H-3,1-benzoxazin-4-on werden kontinuierlich in enem Stickstoffstrom bei 450°C innerhalb 10 Minuten verdampft und bei gleicher Temperatur durch einen Röhrenofen mit 9,47 g Kaliumfluorid geleitet. Das Destillat wird mit Petrolether gewaschen, wobei man 3,1 g eines Gemisches vom Fp. 139 bis 147°C aus 2,39 g (60,6 % d. Th.) 5-Fluor-2-phenyl-4H-3,1-benzoxazin-4-on und 0,71 g (16,9 % d. Th.) der 5-Chlor-Verbindung erhält.

**Beispiel 16**

Eine Mischung aus 20 g 5-Chlor-2-phenyl-4H-3,1-benzoxazin-4-on, 6 g des nach Beispiel 3a vorbehandelten Kaliumfluorids und 0,3 g Natriumjodid wird 3 Stunden bei 330°C gerührt. Bei de anschließenden Destillation über eine beheizte Brücke erhält man 15,4 g eines fast farblosen Destillats vom Fp. 162 bis 164°C, bestehend aus 15,29 g (81,7 % d. Th.) 5-Fluor-2-phenyl-4H-3,1-benzoxazin-4-on und 0,11 g (0,6 %) des nicht umgesetzten Ausgangsmaterials.

**Patentansprüche**

1. Verfahren zur Herstellung von 5-Fluor-2-phenyl-4H-3,1-benzoxazin4-onen I

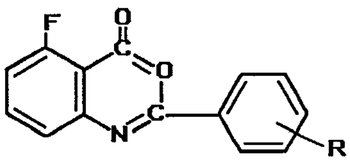

(I)

in der R Wasserstoff, Halogen, $C_1$- bis $C_3$-Alkyl, m- oder p-ständiges Trifluormethyl, Trifluormethoxy, Chlordifluormethoxy, Difluormethoxy, Tetrafluorethoxy, Trifluormethylmercapto oder Chlordifluormethylmercapto bedeutet, dadurch gekennzeichnet, daß man ein entsprechendes 2-Phenyl-CH-3,1-benzoxazin-4-on der allgemeinen Formel II

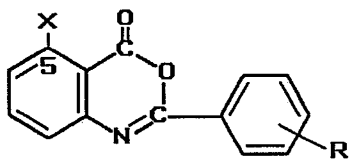

(II)

in der X Chlor, Brom, Jod, Nitro, Mesyl oder Tosyl bedeutet, mit Alkalifluorid unter Ausschluß von Wasser umsetzt.

2. Verfahren nach Anspruch 1, *dadurch gekennzeichnet*, daß man mindestens einen der Rohstoffe mit einem in gasförmige Stoffe zerfallenden Säurehalogenid vorbehandelt.

3. Verfahren nach Anspruch 2, *dadurch gekennzeichnet*, daß man die Vorbehandlung bei einer Temperatur bis 120°C vornimmt.

4. Verfahren nach Anspruch 2, *dadurch gekennzeichnet*, daß man die Umsetzung in Gegenwart des Säurehalogenids vornimmt.

5. Verfahren nach Anspruch 1 oder 2, *dadurch gekennzeichnet*, daß man als Säurehalogenid Thionylchlorid verwendet.

6. Verfahren nach einem der Ansprüche 1 bis 5, *dadurch gekennzeichnet*, daß man Kaliumfluorid als Alkalifluorid verwendet.

7. Verfahren nach einem der Ansprüche 1 bis 6, *dadurch gekennzeichnet*, daß man die Umsetzung in einem Lösungsmittel bei einer Temperatur zwischen 150 und 250°C vornimmt.

8. Verfahren nach einem der Ansprüche 1 bis 7, *dadurch gekennzeichnet*, daß man ein aliphatisches Sulfon als Lösungsmittel verwendet.

9. Verfahren nach einem der Ansprüche 1 bis 8, *dadurch gekennzeichnet*, daß man die Umsetzung in Gegenwart eines löslichkeitsvermittelnden Katalysators vornimmt.

10. Verfahren nach einem der Ansprüche 1 bis 9, *dadurch gekennzeichnet*, daß man die Umsetzung in Gegenwart eines Metalljodids und/oder von Jod durchführt, die Temperatur bei der Umsetzung 160 bis 280°C, insbesondere 180 bis 260°C beträgt und daß man das Metalljodid bzw. Jod während der Umsetzung zusetzt.

11. Verfahren nach Anspruch 1 bis 5, *dadurch gekennzeichnet*, daß man die Umsetzung im wesentlichen ohne Lösungsmittel bei einer Temperatur oberhalb von 250°C durchführt.

12. Verfahren nach Anspruch 11, *dadurch gekennzeichnet*, daß man die Umsetzung zwischen 300 und 500°C durchführt.

13. Verfahren nach Anspruch 11, *dadurch gekennzeichnet*, daß man die Umsetzung in Gegenwart eines Metalljodids zwischen 300 und 400°C durchführt.

14. Verfahren nach einem der Ansprüche 1 bis 13, *dadurch gekennzeichnet*, daß man das während der Reaktion entstehende 5-Fluor-2-phenyl-4H-3,1-benzoxazin-4-on kontinuierlich aus dem Reaktionsgemisch entfernt.

15. Verfahren nach einem der Ansprüche 1 bis 14, *dadurch gekennzeichnet*, daß man die Umsetzung nach anfänglicher Durchmischung der Reaktanden im wesentlichen ohne Rührung nach der Art eines Backprozesses durchführt.

16. Verfahren nach einem der Ansprüche 1 bis 15, *dadurch gekennzeichnet*, daß man die Umsetzung in einem Röhrenreaktor durchführt.

**Claims**

1. A process for the preparation of a 5-fluoro-2-phenyl-4H-3,1-benzoxazin-4-one I

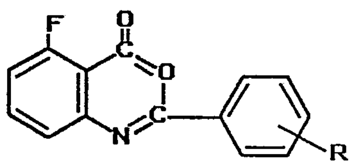

(I)

where R is hydrogen, halogen, $C_1$-$C_3$-alkyl, or trifluoromethyl, trifluoromethoxy, chlorodifluoromethoxy, difluoromethoxy, tetrafluoroethoxy, trifluoromethylmercapto or chlorodifluoromethylmercapto in the m- or p-position, wherein a corresponding 2-phenyl-4H-3,1-benzoxazin-4-one of the general formula II

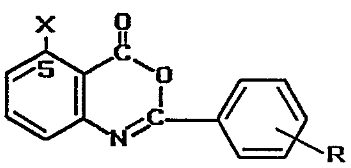

(II)

where x is chlorine, bromine, iodine, nitro, mesyl or tosyl, is reacted with an alkali metal fluoride in the absence of water.

2. A process as claimed in claim 1, wherein one or more of the raw materials are pretreated with an acid halide which decomposes to give gaseous substances.

3. A process as claimed in claim 2, wherein the pretreatment is carried out at no higher than 120°C.

4. A process as claimed in claim 2, wherein the reaction is carried out in the presence of the acid halide.

5. A process as claimed in claim 1 or 2, wherein thionyl chloride is used as the acid halide.

6. A process as claimed in any of claims 1 to 5, wherein potassium fluoride is used as the alkali metal fluoride.

7. A process as claimed in any of claims 1 to 6, wherein the reaction is carried out in a solvent at from 150 to 250°C.

8. A process as claimed in any of claims 1 to 7, wherein an aliphatic sulfone is used as the solvent.

9. A process as claimed in any of claims 1 to 8, wherein the reaction is carried out in the presence of a catalyst which acts as a solubilizer.

10. A process as claimed in any of claims 1 to 9, wherein the reaction is carried out in the presence of a metal iodide and/or iodine, the temperature of the reaction is 160 - 280°C, in particular 180 - 260°C, and the metal iodide and/or iodine are added during the reaction.

11. A process as claimed in claim 1 to 5, wherein the reaction is carried out essentially in the absence of a solvent, at above 250°C.

12. A process as claimed in claim 11, wherein the reaction is carried out at from 300 to 500°C.

13. A process as claimed in claim 11, wherein the reaction is carried out in the presence of a metal iodide, at from 300 to 400°C.

14. A process as claimed in any of claims 1 to 13, wherein the 5-fluoro-2-phenyl-4H-3,1-benzoxazin-4-one formed during the reaction is removed continuously from the reaction mixture.

15. A process as claimed in any of claims 1 to 14, wherein, after initial thorough mixing of the reactants, the reaction is carried out essentially without stirring, by a type of baking process.

16. A process as claimed in any of claims 1 to 15, wherein the reaction is carried out in a tube reactor.

**Revendications**

1. Procédé de préparation de 5-fluoro-2-phényl-4H-3,1-benzoxazine-4-ones I

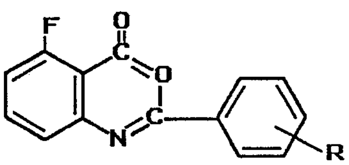

(I)

dans laquelle R désigne un atome d'hydrogène ou d'halogène, un radical alkyle en $C_1$ à $C_3$ ou un groupe trifluoro-méthyle, trifluoro-méthoxy, chloro-difluoro-méthoxy, di-fluoro-méthoxy, tétrafluor-éthoxy, trifluoro-méthyl-mercapto ou chloro-difluoro-méthyl-mercapto en position méta ou para, caractérisé en ce que l'on fait réagir une 2-phényl-4H-3,1-benzoxazine-4-one appropriée, de la formule générale II

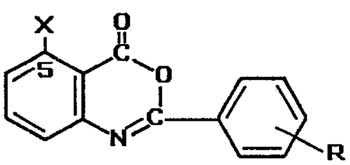

(II)

dans laquelle X désigne un atome de chlore, de brome ou d'iode ou un groupe nitro, mésyle ou tosyle, à l'abri de l'eau avec un fluorure de métal alcalin.

2. Procédé suivant la revendication 1, caractérisé en ce que l'on soumet au moins l'un des composés de départ à un traitement préalable avec un halogénure d'acide se décomposant en produits gazeux.

3. Procédé suivant la revendication 2, caractérisé en ce que ce prétraitement est réalisé à une température inférieure ou égale à 120°C.

4. Procédé suivant la revendication 2, caractérisé en ce que la réaction est réalisée en présence de l'halogénure d'acide.

5. Procédé suivant la revendication 1 ou 2, caractérisé en ce que l'halogénure d'acide est le chlorure de thionyle.

6. Procédé suivant l'une des revendications 1 à 5, caractérisé en ce que le fluorure de métal alcalin est le fluorure de potassium.

7. Procédé suivant l'une des revendications 1 à 6, caractérisé en ce que la réaction est réalisée dans un solvant à une température comprise entre 150 et 250°C.

8. Procédé suivant l'une des revendications 1 à 7, caractérisé en ce que le solvant est une sulfone aliphatique.

9. Procédé suivant l'une des revendications 1 à 8, caractérisé en ce que la réaction est réalisée en présence d'un catalyseur favorisant la solubilité.

10. Procédé suivant l'une des revendications 1 à 9, caractérisé en ce que la réaction est réalisée à une température comprise entre 160 et 280°C et en particulier entre 180 et 260°C en présence d'un iodure de métal et(ou) d'iode, qui est ajouté au cours de la réaction.

11. Procédé suivant les revendications 1 à 5, caractérisé en ce que la réaction est réalisée essentiellement en l'absence d'un solvant à une température supérieure à 250°C.

12. Procédé suivant la revendication 11, caractérisé en ce que la réaction est réalisée entre 300 et 500°C.

13. Procédé suivant la revendication 11, caractérisé en ce que la réaction est réalisée entre 300 et 400°C en présence d'un iodure de métal.

14. Procédé suivant l'une des revendications 1 à 13, caractérisé en ce que la 5-fluoro-2-phényl-4H-3,1-benzoxazine-4-one, qui se forme pendant la réaction, est éliminée en continu du mélange réactionnel.

15. Procédé suivant l'une des revendications 1 à 14, caractérisé en ce que, après le mélange initial des réactifs, la réaction est poursuivie pratiquement sans agitation, à la manière d'un procédé de cuisson.

16. Procédé suivant l'une des revendications 1 à 12, caractérisé en ce que la réaction est réalisée dans un réacteur tubulaire.